# EUROPEAN PATENT APPLICATION

(11) **EP 3 029 463 A1**
(43) Date of publication of application: **08.06.2016**
(21) Application number: 16151842.8
(22) Date of filing: 29.11.2012
(51) Int. Cl.: G01N 33/558, G01N 33/487, A61B 5/00, A61B 3/10, G01N 21/78, G01N 21/80, A61B 10/00, G01N 21/84, A61B 5/145

(54) **DRY EYE DIAGNOSTIC**

(30) Priority: 30.11.2011 US 201161564908 P
(62) Divisional of application: 12854447.5
(71) Applicant: DiagnosTear Ltd, 46344 Herzliya (IL)
(72) Inventor: EILAT, Eran, 46344 Herzliya (IL); DAVID, Robert, Atlanta, GA 30319 (US)
(74) Representative: Cordina, Kevin John

(57) **Abstract**

The present invention discloses a tear analyzing strip (TAS) for measuring dry eye syndrome in a patient. The TAS comprises an elongated body comprising a proximal portion and a distal portion interconnected by a main longitudinal axis. The distal portion comprises at least one tear channel comprising at least one tear receiving portion in fluid communication with an eye of the patient such that tears sampled from the eye into the channel. Reagent pads disposed along the length of the distal portion. The reagent pad is in fluid communication with the tear channel such that tears flow through the tear channel towards the reagent pads, thereby progressively wetting the reagent pads. Each of the reagent pads comprises an effective measure of a reagent capable of biological, physical, or chemical activation by the tears or a component thereof and of providing a visible indication of the activation.

## Description

### FIELD OF THE INVENTION

The present invention generally relates to diagnostic methods and devices and in particular to method and devices for diagnosing dry eye syndrome.

### BACKGROUND OF THE INVENTION

The term "dry eye syndrome" (DES) describes conditions characterized by a disruption of the homeostasis of the ocular surface, as a result of reduced tear production, and/or increased tear evaporation, and/or changes in the integrity of the ocular surface and/or the tear structure (in any of its layers, aqueous, mucine or lipid). The condition is associated with symptoms of ocular discomfort and irritation that can seriously affect the ability of the person suffering from DES to conduct a normal life. Dry eye is one of the most often encountered conditions in ophthalmology practice. It is estimated that 17% to 30% of all people experience dry eye syndrome at some point during their life. Women are more likely to suffer from DES than men are. DES is also a common chronic disease in the elderly population as well. One of the main characteristics of DES is the lack of firm correlation between the level of the ocular surface damage (as evaluated by various objective tests) and that of the ocular symptomatology. Patients with minimal damage may have extensive complaints, while others with severe damage to the conjunctival and corneal surface may only complain of mild discomfort.

One of the parameters for known in the art for assessing the tear production is performing quantitative measurement of the tear. Such a method was introduced by Schirmer in 1903 (Schirmer, O. "Studien zur Physiologie und Pathologie der Tranenabsonderung und Tranenabfluss" Graefes Arch Clin Exp, Ophthalmol 1903, 56, 197-291). The Schirmer Tear Test is performed as follows (Schirmer, O., Studien zur Physiologie und Pathologie der Tranenabsonderung und Tranenabfluss, Graefes Arch Clin Exp, Ophthalmol 56:197-291, 1903). A notched test strip is bent at the notch (∼120° bend). The rounded end of the

According to Schirmer's method, a paper strip is used to measure the amount of tears produced over a period of five minutes. The strip is placed at the junction of the middle and lateral thirds of the lower eyelid, between the eyeball and the lid. The test is done under ambient light. The patient is instructed to look forward and to blink normally during the course of the test. Wetting of more than 10 mm of the paper in 5 minutes is taken to indicate that the eye produces normal quantity of tears. The specificity (the ability of the test to identify negative results) of Schirmer method is usually around 90%. The Schirmer test provides true positive results when the wetting is less the 5 mm and true negative results when the level of wetting is above 10 mm and may provide false positive results when the level of wetting is between 5 mm and 10 mm. When the level of wetting is between 5 mm and 10 mm the patient is suspected to have DES, but the results cannot be considered conclusive.

Recent researches have identified additional parameters that play important roles in the tear homeostasis and lubrication of the ocular surface. Ocular disorders may cause a change in the pH, osmolarity, and temperature of the tear film or surface of the eye as well as change in the tear film concentration of substances such as acid-lactic, glucose, lipids, hormones, gases, enzymes, inflammatory mediators, plasmin, albumin, lactoferrin, creatinin, proteins and other physical characteristics of the eye. More Specifically, tear osmolarity, the protein composition of the tear-film, lysozyme and lactoferin have all been shown to be involved in the delicate balance of normal tear function. The level of cellular function of the goblet cells of the conjunctiva, which secrete the mucine found in the tear film, is known to contribute as well. In addition, dysfunction of the Meybomian Glands has been shown to be important in the etiology of DES. Laboratory techniques for measuring each of these parameters are well-known in the art.

TearLab is a commercially available instrument that measures tear osmolarity by using a pen designed to facilitate tear fluid collection collected directly from the eyelid margin. The TearLab Osmolarity test utilizes a temperature corrected impedance measurement to provide an indirect assessment of osmolarity. After applying a lot-specific calibration curve, the osmolarity is calculated.

US patent 7134998 to Endo et al discloses a tear secretion quantity examination system comprising a moisture evaporation quantity detection unit for detecting the amount of moisture evaporation a subject's eye with a humidity sensor and an operation unit for computing evaluation parameters of the tear secretion quantity based on a detection signal of the moisture evaporation quantity detection unit.

US patent 7129717 to Donsky discloses systems and methods for allowing a rapid, inexpensive estimate of tear film osmolarity that can be made either at home or in the clinic by using a contact lens as a to collect the tear film.

The article "Tear Osmolarity Variation in the Dry Eye" (Farris, R.; Stuchell, R. N.; Mandel, I. D., Trans. Am. Ophthalmol. Soc. 1986, 84,250-268) reports the variations of tear osmolarity in DES patients and in age- and sex-matched normal volunteers.

The article "Diagnostic Implications of Tear Protein Profiles" (Mackie, I. A.; Seal, D. V., Br. J. Ophthalmol. 1984, 68, 321-324) reports the results of ELISA (enzyme-linked immunosorbent assay) measurements of the concentrations of lysozyme, lactoferrin, ceruloplasmin, IgA, and IgG in tears.

The article "Quantitative Tear Lysozyme Assay: a New Technique for Transporting Specimens" (Seal, D. V.; Mackie, I. A.; Coakes, R. L.; Farooqi, B., Br. J. Ophthalmol. 1980, 64, 700-704) discloses a method for assaying the concentration of tear lysozyme using elutes of tear fluid collected on paper discs.

Dry eye syndrome can be treated by avoidance of exacerbating factors, tear stimulation and supplementation, increasing tear retention, eyelid cleansing, and treatment of eye inflammation. Proper diagnosis of the syndrome is thus important for timely treatment, but an inexpensive, reliable method for diagnosing dry eye syndrome remains a long felt yet unmet need.

### SUMMARY OF THE INVENTION

The present invention relates to the field of diagnostic methods and devices for diagnosing dry eye syndrome.

It is one object of the present invention to disclose a tear analyzing strip for measuring dry eye syndrome in a patient, the TAS comprises: an elongated body comprising a proximal portion and a distal portion interconnected by a main longitudinal axis; the distal portion comprising: at least one tear channel comprising at least one tear receiving portion in fluid communication with the junction of the middle and lateral thirds of the lower eyelid of the patient such that tears sampled from the eye into the channel; at least one reagent pad disposed along the length of the distal portion, the at least one reagent pad in fluid communication with the tear channel such that the tears flow through the tear channel towards the at least one reagent pad, thereby progressively wetting the at least one reagent pad; each of the at least one reagent pad comprising an effective measure of at least one reagent capable of biological, physical, or chemical activation by the tears or a component thereof and of providing a visible indication of the activation.

It is a further object of the invention to disclose the TAS which at least a portion of the at least one pad is adapted to detect the amount of tears produced by the eye according to the progress of the tear migration front.

According to another preferred embodiment of the present invention the TAS as defined above wherein at least a portion of the at least one reagent pad is adapted to measure at least one characteristics of the tears.

According to another preferred embodiment of the present invention the TAS as defined above wherein the at least one characteristic of the tears is selected from the group consisting of (a) concentration of at least one tear component chosen from the group consisting of lactoferrin, lysozyme, immunoglobin, cytokines, at least one predetermined protein, electrolytes and growth factors; (b) osmolarity; (c) viscosity and surface tension; and (d) pH.

According to another preferred embodiment of the present invention the TAS as defined above, wherein the proximal portion comprises an elongated support having a handling portion located at the proximal end.

According to another preferred embodiment of the present invention the TAS as defined above, wherein the proximal end of the elongated body comprising a handle.

It is a further object of the invention to disclose the TAS as defined above, wherein the TAS is made of material selected from the group consisting of paper, filter paper, and any combination thereof.

It is a further object of the invention to disclose TAS as defined above, wherein the visual indication is a color change, and wherein the strip is provided with a scale index of the color change.

It is yet another object of the present invention to disclose a method for detecting dry eye syndrome in a patient, comprising: providing a TAS ;placing the TAS at the junction of the middle and lateral thirds of the lower eyelid, of the patient; collecting an effective volume of tears from an eye of a patient by means of the tear receiving portion; detecting at least one of the following: the migration distance of the tear migration front after a predetermined time; the time for the migration front to reach a certain distance; the amount of tears produced according to the measured tear migration front; and, at least one characteristic of the tears.

According to another preferred embodiment of the present invention the method for detecting dry eye syndrome in a patient, wherein the step of detecting comprises a step of detecting at least one characteristic of the tears selected from the group consisting of (a) concentration of at least one tear component chosen from the group consisting of lactoferrin, lysozyme, immunoglobin, cytokines, at least one predetermined protein, electrolytes and growth factors; (b) osmolarity; (c) viscosity and surface tension; and (d) pH.

### BRIEF DESCRIPTION OF THE DRAWINGS

In order to better understand the invention and its implementation in a practice, a plurality of embodiments will now be described, by way of non-limiting example only, with reference to the accompanying drawings, in which
**Fig. 1** illustrates a schematic block diagram of an embodiment of a tear analyzer strip (TAS);
**Fig. 2** illustrates a flowchart diagram of a second method for using a tear analyzer strip in accordance with some exemplary embodiments of the disclosure; and,
**Fig. 3** illustrates an exemplary urine stick as known in the prior art.

### DETAILED DESCRIPTION OF THE PREFERRED EMBODIMENTS

In the following description, various aspects of the invention will be described. For the purposes of explanation, specific details are set forth in order to provide a thorough understanding of the invention. It will be apparent to one skilled in the art that there are other embodiments of the invention that differ in details without affecting the essential nature thereof. Therefore the invention is not limited by that which is illustrated in the figure and described in the specification, but only as indicated in the accompanying claims, with the proper scope determined only by the broadest interpretation of the claims.

The term "multi functional diagnosis" refers herein after to a diagnosis which is based on a plurality of characteristics.

The term "effective volume," when used to described tears collected in some embodiments of the invention herein disclosed, refers to a volume large enough to provide a definitive result when subjected to a particular chemical or physical test. Thus, the "effective volume" will depend on the particular test being performed.

According to some embodiments a Tear Analyzing Strip (TAS) is provided. The TAS comprises one or more chemical pads containing reagents which, upon contact with tears, undergo a chemical reaction as a result of which a color change is observed. The diagnosis may be made after a predefined time, e.g. after completion of the chemical reaction. The diagnosis is based on comparing the colors of the chemical reagent pad to a reference table of colors. The reference table of colors may comprise one or more colors wherein each of the color represents one or more characteristics for diagnosing DES. Such characteristics may be, but not limited to, (a)the concentration of at least one substance the concentration of which is known to correlate with DES (non-limiting examples include lactoferrin, lysozyme, immunoglobulin, cytokines, and growth factors), the concentration of at least one predefined protein level, glucose and electrolyte (such as sodium, potassium etc) (b)osmolarity, (c)viscosity and surface tension and (d) pH.

Methods for colorimetric determination of the concentrations of substances known to correlate with DES are known in the art. Examples of disclosures of these methods, all of which are incorporated herein in their entirety, can be found in the following references. Methods for colorimetric determination of lysozyme and lactoferrin are disclosed in *"*Clinical application of a homogeneous colorimetric assay for tear lysozyme," (1999, Vol. 7, No. 1, Pages 7-15 Andres J. Klaeger, Vicky Cevallos, Mark D. Sherman, John P. Whitcher and Richard S. Stephens) and U.S. Pat. No 3834991

A method for colorimetric determination of immunoglobin is disclosed in E.P Pat. No.0442231.

A method for colorimetric determination of pH is disclosed in The challenge of dry eye diagnosis"by Dove press-Clinical Opththalmology

A method for estimating the viscosity of a liquid in a metering system, such as a liquid transported by a metering system on a diagnostic analyzer is disclosed in US Pat. No. 2011252872

A novel device and method for quickly assessing the osmolarity of a liquid in a semi-quantitative manner is disclosed in "Method For Quick Assessment Of Osmolarity" of the inventor Eran Eilat.

In the above mentioned the devise comprises an area of the support impregnated with vesicles(a continuous integument containing a liquid, the integument may also contain a coloring agent, a swelling agent, and possibly inert ingredients) in it. Vesicles with diameters of less than 0.5µm that will allow light to pass through them and be reflected from the underlying support, while vesicles with diameters greater than 0.5µm will absorb light so that the underlying color of the support will be hidden. In the dry state the vesicles are of medium size; some of the color of the underlying support will be seen. If exposed to a liquid of high osmolarity, the vesicles will shrink and the apparent color of the underlying support will become more intense. If exposed to a liquid of low osmolarity, the vesicles expand, absorb visible light, and the apparent color of the underlying support will become less intense.

In order to measure the osmolarity of the tear film of the eye to assess or diagnose dry eye syndrome, tears would be absorbed from the eye, in the same manner as is commonly done in the Schirmer test, on to an absorbent support such as abosorbent paper where at least one area of the absorbent support is impregnated with vesicles of a multilayer polymer produced in a manner similar to US 5260069 but where said vesicles contains a coloring agent such as a dye in solution at an osmolarity of 300 mOs. If the tear osmolarity is relatively high (e.g. 350 mOs) then the vesicles will shrink and the absorbing paper will remain its original color. If the tear film osmolarity is relatively low (e.g. 280 mOs), then the vesicles will burst and the coloring matter will be released, hence leaving color (e.g. red) on the absorbent support. The lower the osmolarity, the more intense the color will be (the redder the support will appear).

These methods are provided as non-limiting examples; any method known in the art that is suitable for use with a test strip maybe used in the invention herein disclosed." According to some embodiments, the TAS comprises an elongated body for measuring the amount of moisture from the tears that are produced over a period of time and thus provide a measurement of the quantitative production of tear as in the Schirmer test.

The TAS may also be useful for collecting an amount of tear fluid sufficient for performing a medical diagnosis based on the relevant characteristics of the tears. The TAS thus can provide qualitative, quantitative, semi-quantitative and multi-factorial diagnosis.

Collecting an effective volume of tears from at least one eye of a patient measuring at least one physical or chemical characteristic of the tears is necessary to ensure accurate evaluation of the outcome of the reaction between the tears and the reagent on the pad, and thus improves the assessment of the level of damage of the ocular surface.

In some embodiments of invention herein disclosed, tears are collected from the eye by attaching the TAS to the eyelid between the lower lid and the eyeball. In other embodiments, the tears are first collected from the lower eyelid to an elongated tear channel which is disposed along the length of the tear analyzing strip.

The TAS may be used for evaluating the course of DES in patients over time, as well as for evaluating the patient's response to treatment.

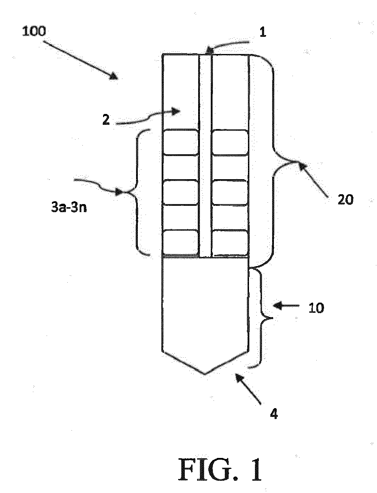

Reference is made now to Fig.1 which illustrates a schematic block diagram of an embodiment of a TAS (100). In some embodiments, a tear analyzing strip (TAS) (100) is provided. The TAS comprises an elongated body characterized by a proximal portion (10) and a distal portion (20) interconnected by a main longitudinal axis; the distal portion (20) comprises (a) at least one tear channel (2) having at least one opening in fluid communication with the patient's eye such that tears sampled from the patient's eye flow through the opening into the channel (2); and (b) at least one reagent pad (3a-3n) comprising an effective measure of a reagent that can be biologically, physically or chemically activated by means of the tears to yield a visible indication of reaction, the reagent pads in fluid connection with the tear channel (2) and disposed along the length of the TAS such that tears flow through the tear channel (2) towards the reagent pads (3a-3n) and progressively wet them. The time for the n^{th} reagent pad to be wetted (or alternatively the number of reagent pads wetted in a predetermined time) indicates the amount of tears being produced according to the rate of advancement of the tear migration front.

The TAS may be of a filter paper strip or any type of paper in any appropriate shape or configuration such as a 2d body, 3d body or any combination thereof.

In various non-limiting exemplary embodiments of the invention, the TAS is used for i) collecting a first effective volume of tears from an eye of a patient; ii) measuring the migration distance of the tear migration front after a predetermined time; iii) measuring the time for the tear migration front to pass through a given distance; or iv) measuring the amount of tears produced according to location of the tear migration front.

The reagent pads 3a-3n may comprise a reagent or a substance or a compound that produces a chemical reaction when being in contact with the fluid of the tears.

The reaction may be used to identify or to measure characteristics of the tears. In some embodiments of the invention, each reagent pad is used to identify a different characteristic. In various embodiment of the invention, the characteristic may be chosen from (a) concentration of at least one tear component chosen from the group consisting of lactoferrin, lysozyme, immunoglobin, cytokines, at least one predetermined protein, electrolytes and growth factors; (b) osmolarity; (c) viscosity and surface tension; and (d) pH.

In some embodiments, the TAS has a handling portion 132 located at the proximal portion 10. In such a case, the reagent pads 3a-3n are in fluid communication with the elongated tear channel 2 located along the distal portion 20. The distal portion 20 comprises a distal end 1 which may be attached to the patient's eyelid between the lower lid and the eyeball.

In some other embodiments, the TAS may further include an elongated support having a handling located at the proximal portion 10. In some embodiments, the elongated support is a stick such as a plastic stick or a wooden stick.

The TAS 100 may collect a sufficient amount of tears from the lower eyelid and may determine dry eye syndrome according to one or more of said characteristics of the tear fluid.

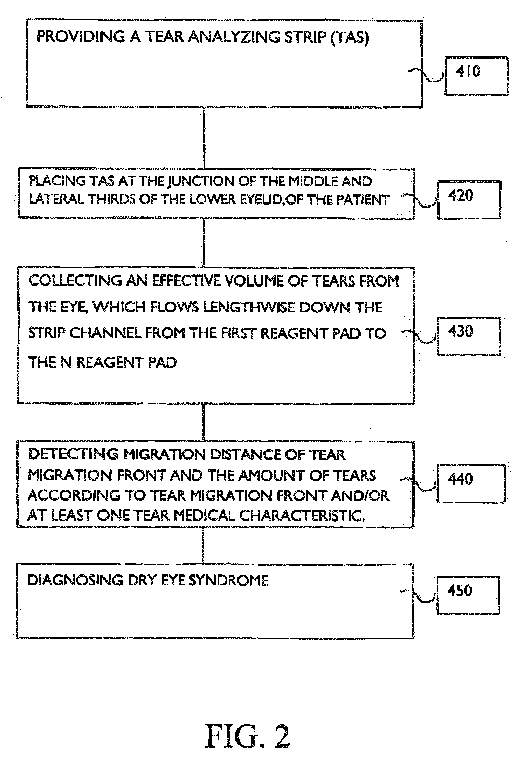

Reference is now made to Fig. 2 illustrates a flowchart diagram of a method for using the invention herein disclosed to evaluate the level of DES.

In the first step of the method (410) a TAS as defined in any of the above is provided.

In the second step (420) the TAS is placed at the junction of the middle and lateral thirds of the lower eyelid, of the patient.

An effective volume of tears is then collected (430) from the patient's eye by the paper strip which flows within the elongated tear channel to the successive reagent pads.

In some embodiments of the method, the migration distance of the tear migration front is detected (440) and the amount of tears produced is determined according to the tear migration front; in some embodiments, at least one tear characteristic is measured from the effective volume of tears by means of the aforementioned reaction with reagent contained in a pad with which the tears interact. In various embodiment of the invention, the characteristic may be chosen from (a) concentration of at least one tear component chosen from the group consisting of lactoferrin, lysozyme, immunoglobin, cytokines, growth factors at least one predetermined protein, glucose and electrolytes ; (b) osmolarity; (c) viscosity and surface tension; and (d) pH.

The determination of the value of the characteristic may be made by means of a paper strip. Such means may be a scale index which consists of visual scale of variety colors.

Dry eye syndrome is then diagnosed (450) from the results of one or more reactions between the tears (or components therein) and the reagents in the reagent pads and from the migration distance of tear migration front.

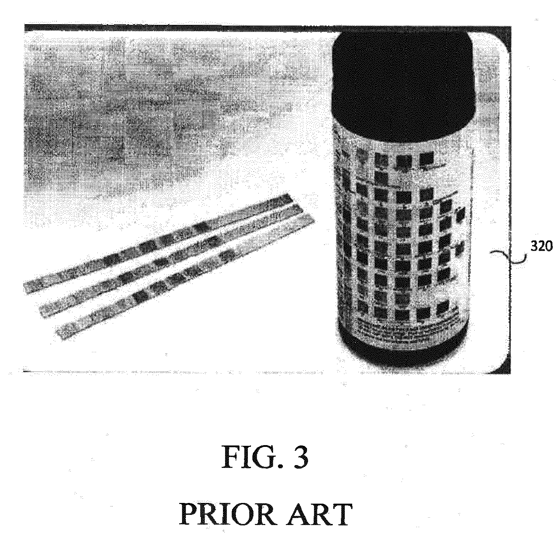

Reference is now made to FIG. 3, which illustrates a typical scale index of a type known in the art that can be used to compare the color change of the reagent pads with colors indicating the degree of completion of a particular reaction.

Fig. 3 illustrates an exemplary urine stick as known in the prior art. The urine stick may be used to determine the pH, specific gravity, protein, glucose, ketone, bilirubin, urobilinogen, blood, leukocyte, and nitrite levels of an individual's urine. It consists of a reagent stick-pad, which is immersed in a fresh urine specimen and then withdrawn. After predetermined times the colors of the reagent pad are compared to standardized reference charts 320. The urine dipstick offers an inexpensive and fast method to perform screening urinalyses, which help in identifying the presence of various diseases or health problems.

Further aspects of the invention are set forth below as numbered clauses:
1. A tear analyzing strip (TAS, 100) for measuring dry eye syndrome in a patient, wherein said TAS comprises: an elongated body comprising a proximal portion (10) and a distal portion (20) interconnected by a main longitudinal axis; said distal portion (20) comprising: at least one tear channel (2) comprising at least one tear receiving portion in fluid communication with an eye of said patient such that tears sampled from said eye into said channel; at least one reagent pad disposed along the length of said distal portion, said at least one reagent pad in fluid communication with said tear channel such that said tears flow through said tear channel towards said at least one reagent pad, thereby progressively wetting said at least one reagent pad; each of said at least one reagent pad comprising an effective measure of at least one reagent capable of biological, physical, or chemical activation by said tears or a component thereof and of providing a visible indication of said activation.
2. The TAS of clause 1, wherein at least a portion of said at least one pad is adapted to detect the amount of tears produced by said eye according to the progress of the tear migration front.
3. The TAS of clause 1, wherein at least a portion of said at least one reagent pad is adapted to measure at least one characteristics of said tears.
4. The TAS of clause 3, wherein said at least one characteristic of said tears is selected from the group consisting of (a) concentration of at least one tear component chosen from the group consisting of lactoferrin, lysozyme, immunoglobin, cytokines, growth factors at least one predetermined protein level, glucose electrolytes and (b) osmolality; (c) viscosity and surface tension; and (d) pH.
5. The TAS of clause 1, wherein said proximal portion comprises an elongated support having a handling portion located at the proximal end.
6. The TAS of clause 1, wherein said proximal end of said elongated body comprising a handle.
7. The TAS of clause 1, wherein said TAS is made of material selected from the group consisting of paper, filter paper, and any combination thereof.
8. The TAS of clause 1 , wherein said visual indication is a color change, and wherein said strip is provided with a scale index of said color change.
9. A method for detecting dry eye syndrome in a patient, comprising: providing a TAS according to clause 1 ; placing said TAS at the junction of the middle and lateral thirds of the lower eyelid,of said patient; collecting an effective volume of tears from an eye of a patient by means of said tear receiving portion; detecting at least one of the following: the migration distance of the tear migration front after a predetermined time; the time for the migration front to reach a certain distance; the amount of tears produced according to the measured tear migration front; and, at least one characteristic of said tears.
10. The method of clause 9, wherein said step of detecting comprises a step of detecting at least one characteristic of said tears selected from the group consisting of (a) concentration of at least one tear component chosen from the group consisting of lactoferrin, lysozyme, immunoglobin, cytokines growth factors, at least one predetermined protein levels, glucose and electrolytes ; (b) osmolality; (c) viscosity and surface tension; and (d) pH.
11. A multi-functional dry eye syndrome diagnostic comprising: a first absorbent support; a second absorbent support; a third absorbent support having a color; a first dry reagent pad on the first absorbent support, wherein the first dry reagent pad is sufficiently designed to measure a first concentration of a first tear analyte that is a protein, wherein the first concentration of the first tear analyte produces a first visible color that correlates to a first characteristic of dry eye syndrome; a second dry reagent pad on the second absorbent support, wherein the second dry reagent pad is sufficiently designed to measure a second concentration of a second tear analyte that is an enzyme, wherein the second concentration of the second tear analyte produces a second visible color that correlates to a second characteristic of dry eye syndrome; and a third dry reagent pad on the third absorbent support, wherein the third dry reagent pad is impregnated with vesicles comprising a swelling agent, and wherein a size change of the vesicles, after reacting with tear fluid, produces a third visible color that correlates to tear osmolarity.
12. The diagnostic of clause 11 wherein the first tear analyte is one or more of lactoferrin, mucin, a lipid, an immunoglobulin, an albumin, a growth factor, lysozyme, or plasmin.
13. The diagnostic of clause 11 wherein the first absorbent support, the second absorbent support, and the third absorbent support are filter paper.
14. The diagnostic of clause 11 wherein the first absorbent support, the second absorbent support, and the third absorbent support are paper.
15. The diagnostic of clause 11 further comprising: a fourth absorbent support; and a fourth dry reagent pad on the fourth absorbent support, wherein the fourth dry reagent pad is sufficiently designed to measure a third concentration of a third tear analyte that is an electrolyte, wherein the third concentration of the third tear analyte produces a fourth visible color that correlates to a fourth characteristic of dry eye syndrome.
16. A method for determining a concentration of multiple analytes in tear fluid comprising: providing the multi-functional diagnostic of clause 1; wetting the first dry reagent pad with tear fluid so as to create a first moistened reagent pad; wetting the second dry reagent pad with tear fluid so as to create a second moistened reagent pad; wetting the third dry reagent pad with tear fluid so as to create a third moistened reagent pad; observing the first moistened reagent pad so as to determine whether a reaction with the first tear analyte, a derivative of the first tear analyte, or an indicator compound for the first tear analyte produces a first visible color; observing the second moistened reagent pad so as to determine whether a reaction with the second tear analyte, a derivative of the second tear analyte, or an indicator compound for the second tear analyte produces a second visible color; and observing the third moistened reagent pad so as to determine whether the color on the third absorbent support is more or less intense.
17. The method of clause 16 further comprising: comparing the first visible color from the first moistened reagent pad with a standard color chart; and comparing the second visible color from the second moistened reagent pad with a standard color chart.
18. The method of clause 17 wherein comparing the first visible color from the first moistened reagent pad with a standard color chart results in determining the first concentration of the first tear analyte in the tear fluid, and wherein comparing the second visible color from the second moistened reagent pad with a standard color chart results in determining the second concentration of the second tear analyte in the tear fluid.
19. A tear fluid analyzing strip (TAS, 100) for measuring dry eye syndrome in a patient, wherein said TAS comprises: an elongated body comprising a proximal portion (10) and a distal portion (20) interconnected by a main longitudinal axis; said distal portion (20) comprising: at least one tear channel (2) comprising at least one tear receiving portion in fluid communication with an eye of said patient such that tears sampled from said eye flow into said channel; reagent pads disposed along the length of said distal portion, said reagent pads are in fluid communication with said tear channel such that said tears flow through said tear channel directly towards said reagent pads, thereby progressively wetting said reagent pads; each of said reagent pads provide an effective measure of at least one reagent capable of biological, physical, or chemical activation by said tears or a component thereof and of providing a visible indication of said activation.
20. A tear fluid analyzing strip (TAS, 100) for measuring dry eye syndrome in a patient, wherein said TAS comprises: an elongated body comprising a proximal portion (10) and a distal portion (20) interconnected by a main longitudinal axis; said distal portion (20) comprising: at least one tear channel (2) comprising at least one tear receiving portion in fluid communication with an eye of said patient such that tears sampled from said eye flow into said channel; at least one reagent pad disposed along the length of said distal portion, said reagent pad in fluid communication with said tear channel such that said tears flow through said tear channel directly towards said at least one reagent pad, thereby progressively wetting said at least one reagent pad; said reagent pad provides an effective measure of at least one reagent selected from the group consisting of osmolarity, viscosity and a combination thereof, thereby providing a visible indication.
21. The TAS of clauses 19-20, wherein at least a portion of said reagent pads is adapted to detect the amount of tears produced by said eye according to the progress of the tear migration front.
22. The TAS of clauses 19-20, wherein at least a portion of said reagent pads is adapted to measure at least one characteristic of said tears.
23. The TAS of clauses 19-20, wherein said reagent pads provides an effective measure of at least one characteristic of said tears.
24. The TAS of clauses 19-20, wherein said at least one characteristic of said tears is selected from the group consisting of (a) concentration of at least one tear component chosen from the group consisting of lactoferrin, lysozyme, immunoglobin, cytokines, growth factors at least one predetermined protein level, glucose electrolytes and (b) osmolarity; (c) viscosity and surface tension; and (d) pH.
25. The TAS of clauses 19-20, wherein said reagent pads are impregnated with vesicles of a multilayer polymer for measuring said osmolarity of said tear film of said eye.

## Claims

1. A method for detecting dry eye syndrome in a patient, comprising:
providing a TAS configured to measure, by a chemical reaction, at least one characteristic of a volume of tears collected from an eye of a patient selected from the group consisting of:
a) a concentration of at least one tear component selected from the group consisting of: lactoferrin, lysozyme, immunoglobin, cytokines, at least one predetermined protein, electrolytes and growth factors,
b) an osmolarity,
c) a viscosity and a surface tension,
d) a pH, and
e) any combination thereof,
contacting the TAS with the volume of tears collected from the eye of the patient,
detecting a result of the at least one chemical reaction, and
correlating the result of the at least one chemical reaction with a reference table to determine the at least one characteristic of the tears from the eye of the patient.
